# EUROPÄISCHE PATENTSCHRIFT

(11) **EP 2 010 107 B1**
(45) Veröffentlichungstag und Bekanntmachung des Hinweises auf die Patenterteilung: **15.08.2012**
(21) Anmeldenummer: 07724345.9
(22) Anmeldetag: 18.04.2007
(51) Int. Cl.: A61F 2/34, A61F 2/46

(54) **MODULARES HÜFTIMPLANTAT**
MODULAR HIP IMPLANT
IMPLANT MODULAIRE DE LA HANCHE

(30) Priorität: 19.04.2006 DE 202006006349 U
(43) Veröffentlichungstag der Anmeldung: 07.01.2009
(73) Patentinhaber: Brehm, Peter, 91085 Weisendorf (DE)
(72) Erfinder: Brehm, Peter, 91085 Weisendorf (DE)
(74) Vertreter: Grünecker, Kinkeldey, Stockmair & Schwanhäusser
(86) Internationale Anmeldenummer: PCT/EP2007/003408
(87) Internationale Veröffentlichungsnummer: WO 2007/118708

(56) Entgegenhaltungen:
- EP-A- 0 591 594
- EP-A- 1 082 949
- EP-A- 1 195 149
- DE-A- 19 542 116
- FR-A- 2 827 503
- US-A- 5 108 445
- US-A1- 2005 288 793

## Beschreibung

Die Erfindung bezieht sich allgemein auf Hüftimplantate, insbesondere auf ein modulares Hüftimplantat mit einem Grundkörper in Form einer acetabulären Abstützschale zum Befestigen an einem Beckenknochen und mit einer Pfanne zum Aufnehmen einer Hüftgelenkprothese, wobei der Grundkörper am Beckenknochen befestigbar ist und einen Aufnahmebereich für die Pfanne aufweist, wobei die Pfanne eine zu dem Aufnahmebereich komplementäre Außenform aufweist.

Künstliche Hüftgelenke weisen eine in den Oberschenkelknochen einzusetzende Hüftgelenkprothese und eine am Beckenknochen anzubringende Hüftgelenkpfanne auf. Das vom Knie wegweisende Ende der Hüftgelenkprothese ist kugelförmig ausgestaltet, um in die Hüftgelenkpfanne eines Hüftimplantats einzugreifen und mit dieser ein Kugelgelenk zu bilden.

Bietet das Becken nicht mehr ausreichend knöcherne Substanz zur sicheren Verankerung einer einfachen Hüftgelenkpfanne, dann ist eine zusätzliche Abstützung mit Ring- oder Schalenkonstrukten erforderlich. Diese kommen bevorzugt bei Hüftgelenks-Wechseloperationen zum Einsatz, bei denen der tatsächliche Knochenverlust erst intraoperativ nach dem Ausbau der Primärimplantate feststeht. Zu den Ursachen für den Wechsel einer Pfanne zählt u.a. deren Verankerung mit Knochenzement. Auch bei einer erfolgreichen Pfannenrevision muss mit einer späteren nochmaligen Revision gerechnet werden. Neben dem Wiederaufbau von Knochendefekten mit Transplantaten bietet eine einfach handhabbare rein zementfreie Verankerung daher wesentliche Vorteile.

Wesentliche Ziele beim Einbringen einer künstlichen Hüftpfanne, bzw. einer Revisionspfanne sind die:
1. primär stabile Verankerung von Knochentransplantaten,
2. primär stabile Verankerung der Hüftpfanne, bzw. Abstützschale,
3. Rekonstruktion des anatomischen Hüftrotationszentrums,
4. korrekte Kippung der Pfanne in den sogenannten Winkeln Inklination und Anteversion.

Verbindet man die beiden Drehzentren der zwei Hüftgelenke mit einer ersten (Horizontal)Achse, so stellt die Anteversion den Winkel der Pfannenachse gegenüber der ersten (Horizontal-)Achse in einer (Horizontal-)Ebene durch die erste (Horizontal-)achse und eine zweite anterioposteriore (Horizontal-)Achse dar. Die Inklination stellt den Winkel der Pfannenachse gegenüber der ersten (Horizontal-)Achse in einer (Vertikal-)Ebene durch die erste (Horizontal-)achse und eine kraniokaudale (Vertikal-)Achse dar. Im menschlichen Körper weist die Inklination Werte im Bereich von 30° bis 50° auf, während bei der Anteversion Werte im Bereich von 5 bis 25° gemessen werden.

Die Lage des Hüftrotationszentrums bestimmt die Biomechanik des Hüftgelenkes und damit auch die Standzeit der implantierten Endoprothese. Daneben werden der Bewegungsumfang und die Kraftweiterleitung durch die Anteversion und durch die Inklination festgelegt. Bei unkorrekter Wahl der Anteversion kann der Hals der Femurschaftprothese an den Pfannenrand bzw. die Abstützschale stoßen und die Gelenkkugel aus dem Pfannen/Schalen-Konstrukt heraushebeln. Auch bei unkorrekter Inklination mit fehlender Überdachung der Gelenkkugel durch die Hüftpfanne besteht die Gefahr der Luxation des Hüftgelenkes.

Bei der Implantation eines künstlichen Hüftgelenks muss der Operateur auf die individuellen anatomischen Gegebenheiten des zu behandelnden Körpers, inklusive der Skelettstruktur mit Knochendefekten und der Muskel-/Bandsituation des Halteapparates, eingehen können. Die genannten Zusammenhänge erzwingen jedoch häufig einen Kompromiss zwischen primärstabiter Verankerung von Abstützschalen am Knochen, optimaler Lage des Hüftrotationszentrums und korrekten Inklinations- und Anteversionswinkeln. Gerade bei den schwierigen Implantatwechselsituationen, ist bei vollständig zementfreier Versorgung zusammen mit erweiterten Einstellungsmöglichkeiten der Anteversion und Inklination eine einfache Handhabung wünschenswert.

Gängige Hüftimplantate für Revisionen bestehen aus einem schalenförmigen Grundkörper, der in erster Linie eine Abstützfunktion übernimmt. Diese werden in der Regel zementfrei primär stabil am Beckenknochen verankert. In diese Schale kann, unter Beachtung des korrekten Inklinations- und Anteversionswinkels ein pfannenförmiger Einsatz, zementfrei oder zementiert eingebracht werden, der die Kugel der Hüftgelenksprothese aufnimmt.

Die zementierte Verankerung der Pfanne hat den Vorteil, dass diese innerhalb gewisser Grenzen unabhängig in der Schale eingestellt werden kann. Die zusätzliche Einbringung von Knochenzement ist jedoch in vielen Fällen wegen seiner bekannten Nachteile unerwünscht und bietet in der Schalenkonstruktion keinen langlebigen Halt gegen die zwischen Schale und Pfanne auftretenden Schwingungen.

Aus US 5,425,778 ist ein acetabulärer Abstützring bekannt, der die Einstellbarkeit von Anteversion und Inklination einer zementfreien Pfanne erlaubt. Darin wird die Gelenkkugel gefasst und in ihrer Position gehalten zwischen einem innenseitig konkav geformten Abstützring und einem in diesen einschraubbaren zweiten Ring, der auf dem der Gelenkkugel zugewandten Abschnitt ebenfalls konkav ausgebildet ist. Ein Nachteil dieser Lösung besteht darin, dass die Pfanne bei Kippung unter das Randniveau des Abstützringes taucht (US 5,425,778: Fig. 2) und somit der Hals des Femurschaftes an den Rand der Abstützschale stoßen kann. Die Gefahr, dass auch bei dieser Lösung die Gelenkkugel aus der Pfanne herausgehebelt werden kann, erscheint auch nicht durch Ausnehmungen des Abstützringes vermeidbar, weil sie mit Verlust des Innengewindes in demselben verbunden sind. Außerdem kann es vorkommen, dass sich die einzelnen Teile des Abstützrings nach der Operation in Folge stärkerer mechanischer Belastungen relativ zueinander verdrehen, so dass sich eine ungünstige Anteversion und/oder Inklination ergibt. Außerdem kann es schwierig sein, die Anteversion und die Inklination während der Operation korrekt einzustellen.

EP-A-1 082 949 offenbart eine Gelenkspfannenprothese mit einer äußeren Schale und einem Einsatz, der in die äußere Schale eingesetzt werden kann. Im Einsatz und der äußeren Schale befinden sich Bohrungen, durch die Schrauben geführt werden können, um den Einsatz und die äußere Schale miteinander zu verbinden. Die Bohrungen in der äußeren Schale sind so angebracht, dass verschiedene relative Orientierungen zwischen dem Einsatz und der äußeren Schale möglich sind. Dadurch könnte Anteversion und Inklination eingestellt werden.

Die Merkmale des Oberbegriffs des Anspruchs 1 werden in US 5,108,445 offenbart.

Es ist Aufgabe der Erfindung, ein modulares Hüftimplantat zur Verfügung zu stellen, das die Operationstechnik beim Einsetzen eines Hüftimplantats in den zu behandelnden Körper vereinfacht und sich besser an die anatomischen Erfordernisse eines Patienten anpassen lässt.

Eine weitere Aufgabe der Erfindung ist, ein zementfrei am Knochen befestigbares Schalenkonstrukt bereitzustellen, in dem der Lagereinsatz einer Hüftgelenksprothese (wahlweise zementiert oder) zementfrei eingebracht werden kann und das auch bei zementfreier Befestigung die sichere Einstellung von Inklinations- und Anteversionswinkel bei vereinfachter OP-Technik erlaubt.

Noch eine weitere Aufgabe der Erfindung ist es, ein modulares Hüftimplantat mit verbesserter Stabilität bereitzustellen.

Ein modulares Hüftimplantat gemäß der vorliegenden Erfindung umfasst die in Anspruch 1 definierten Merkmale.

Die erfindungsgemäße Lösung ermöglicht eine an die anatomischen Erfordernisse des Patienten angepasste sichere Einstellung der Inklination und/oder der Anteversion der Pfanne bezüglich des Grundkörpers während der Operation, oder bei einer gegebenenfalls erst zu einem späteren Zeitpunkt vorgenommenen Revision. Durch die zusammenwirkenden Führungsmittel können der Grundkörper und die Pfanne auf besonders einfache Weise relativ zueinander positioniert werden, um die Pfanne in dem Grundkörper definiert zur Erzielung einer gewünschten Inklination und/oder Anteversion zu verstellen. Außerdem verbessern die Führungsmittel die Stabilität des Hüftimplantats gegenüber mechanischen Beanspruchungen.

Durch die Vielzahl von Bohrungen in der Pfanne und im Aufnahmebereich, wobei korrespondierende Bohrungen in der Pfanne und dem Aufnahmebereich durch Bewegung der Pfanne entlang der Führungsmittel in Flucht miteinander bringbar sind, werden Befestigungsmittel bereitgestellt, die besonders einfach in der Pfanne und im Grundkörper eingesetzt werden können. Da die Führungsmittel eine Nut und einen Vorsprung umfassen, lassen sich durch einfache Drehung der Pfanne bzw. ihres ersten Teils in dem Grundkörper unterschiedliche Inklinationswinkel und Anteversionswinkel einstellen. Durch das Vorhalten von Mitteln an Pfanne und Grundkörper, die einen Formschluß erreichen, und dabei so angeordnet sind, dass sie bei Bewegung, vorzugsweise Drehung, der Pfanne gegenüber des Grundkörpers eine Verstellung der Neigung entsprechend der unten angeführten Winkelangaben bezüglich des Grundkörpers hervorrufen, ist eine genaue Anpassung auf den individuellen Einzelfall möglich.

In einer vorteilhaften Ausführungsform ist der Aufnahmebereich des Grundkörpers ringförmig, als zweiseitig offene Kugelschale ausgeformt. Durch die dem Becken zugewandte Öffnung kann bei bereits befestigtem Grundkörper Knochenmaterial zur abschließenden Rekonstruktion noch verbliebener Defekte eingebracht werden.

In einer weiteren vorteilhaften Ausführungsform ist die Pfanne als Kugelschale, insbesondere als Halbkugelschale, ausgeformt. Dadurch werden eine Drehung der Pfanne relativ zum Grundkörper und eine große Kontaktfläche zwischen der Pfanne und dem Grundkörper ermöglicht.

Wenn zumindest einige der Bohrungen ein Innengewinde aufweisen, so lässt sich die Pfanne einfach durch Verschraubung in dem Grundkörper endgültig fixieren.

Wenn die Bohrungen unterschiedliche Durchmesser aufweisen, so lässt sich für den Operateur besonders einfach feststellen, welche Bohrungen von Pfanne und Aufnahmebereich miteinander fluchten und zueinander passen.

Geeigneterweise ist die Pfanne durch eine Schraubverbindung durch die Bohrungen im Aufnahmebereich des Grundkörpers fixierbar. Dadurch kann mit geringem Arbeitsaufwand eine stabile Verbindung zwischen der Pfanne und dem Grundkörper geschaffen werden.

In anderen Ausführungsformen der Erfindung ist die Außenseite der Pfanne so ausgebildet, dass die Pfanne an dem Grundkörper über eine Zementschicht befestigbar ist. Eine Befestigung der Pfanne durch eine Zementschicht stellt eine Alternative zur Befestigung der Pfanne mittels einer Schraubverbindung dar.

Gemäß einem Beispiel umfasst ein modulares Hüftimplantat einen Grundkörper zum Befestigen an einem Beckenknochen und mit einer Pfanne zum Aufnehmen einer Hüftgelenkprothese, wobei der Grundkörper an dem Beckenknochen befestigbar ist und einen Aufnahmebereich für die Pfanne aufweist, wobei die Pfanne eine zu dem Aufnahmebereich komplementäre Außenform aufweist und wobei die Pfanne einen ersten Teil mit einer Öffnung und einen zweiten Teil umfasst, wobei der zweite Teil durch die Öffnung des ersten Teils führbar und am Grundkörper befestigbar ist und wobei der zweite Teil dafür ausgelegt ist, den ersten Teil relativ zum Grundkörper zu fixieren.

Durch die Befestigung der Pfanne an dem Grundkörper kann ein Kontakt zwischen dem Hals des Femurschafts und der Befestigung der Pfanne vermieden werden. Die Gefahr eines Auskugelns des künstlichen Hüftgelenks kann so verringert werden. Außerdem kann eine verbesserte Stabilität der Befestigung der Pfanne am Grundkörper erzielt werden, da die Befestigung vom exponierten oberen Rand der Pfanne entfernt ist. Ferner ist die Formgebung des oberen Randes der Pfanne nicht mehr durch die Notwendigkeit, ein Gewinde anzubringen, eingeschränkt.

Geeigneterweise umfassen der Grundkörper und der zweite Teil der Pfanne miteinander korrespondierende Befestigungsmittel, um eine schnelle und sichere Verbindung dieser Komponenten unter operativen Bedingungen zu ermöglichen.

Vorzugsweise umfassen die Befestigungsmittel ein Innengewinde im Grundkörper und ein mit diesem korrespondierendes Außengewinde am zweiten Teil der Pfanne. Dadurch können der zweite Teil der Pfanne und der Grundkörper miteinander verschraubt werden, wodurch eine gegenüber Mikrobewegungen und Schwingungen besonders sichere Langzeitverbindung zwischen Pfanne und Grundkörper erzielt werden kann.

Das Innengewinde im Grundkörper und das mit diesem korrespondierende Außengewinde am zweiten Teil der Pfanne können als Feingewinde ausgebildet sein. Dadurch kann eine besonders stabile Verbindung zwischen dem Grundkörper und dem zweiten Teil der Pfanne geschaffen werden.

Geeigneterweise weist der Grundkörper mindestens eine Bohrung auf, wobei eine Achse der mindestens einen Bohrung parallel zu einer Achse des Innengewindes im Grundkörper ist. In die Bohrung kann ein Werkzeug zum Zusammenbau des Hüftimplantats eingesetzt werden, mit dessen Hilfe der zweite Teil der Pfanne mit dem Grundkörper verschraubt werden kann.

In weiteren Beispielen kann der zweite Teil der Pfanne durch eine Zementschicht am Grundkörper befestigt werden, wodurch ebenfalls eine sehr stabile Verbindung zwischen dem Grundkörper und der Pfanne erreicht werden kann.

Geeigneterweise weist der zweite Teil der Pfanne eine oder mehrere Bohrungen auf, die zum Aufnehmen von Schrauben, die mit dem Beckenknochen verbindbar sind, ausgebildet sind. Wird der zweite Teil der Pfanne am Beckenknochen festgeschraubt, werden die beiden Pfannenteile und der Grundkörper sowohl in ihrer relativen Lage zueinander als auch in ihrer Position relativ zum Beckenknochen fixiert, so dass eine Verschiebung des künstlichen Hüftgelenks bzw. von dessen einzelnen Komponenten durch mechanische Beanspruchungen im wesentlichen ausgeschlossen ist.

Der Aufnahmebereich kann als zweiseitig offene Kugelschale ausgebildet sein, so dass durch die dem Becken zugewandte Öffnung bei bereits befestigtem Grundkörper Knochenmaterial zur abschließenden Rekonstruktion noch verbliebener Defekte eingebracht werden kann.

Geeigneterweise ist der erste Teil der Pfanne als zweiseitig offene Kugelschale ausgebildet. Dadurch weist der erste Teil der Pfanne eine zum Durchführen des zweiten Teils geeignete Öffnung an einer zentralen Stelle auf.

Vorzugsweise ist der erste Teil der Pfanne in mehreren Positionen relativ zum Grundkörper fixierbar. Dadurch kann die Geometrie des Hüftimplantats während der Operation an die anatomischen Gegebenheiten des Patienten angepasst werden. So ist es nicht notwendig, eine Vielzahl unterschiedlicher Hüftimplantate bereitzustellen, aus denen bei der Operation ein passendes ausgewählt werden muss.

In manchen Beispielen können der erste Teil der Pfanne und der Grundkörper eine Einrichtung zum Vorverriegeln des ersten Teils der Pfanne in den mehreren Positionen relativ zum Grundkörper umfassen. Dadurch kann das Hüftimplantat einfacher implantiert werden, da der erste Teil der Pfanne beim Einsetzen des zweiten Teils der Pfanne nicht mehr von Hand festgehalten werden muss.

Die Einrichtung zum Vorverriegeln des ersten Teils der Pfanne kann einen Bajonettverschluss umfassen. Dadurch kann eine leicht herzustellende und unkompliziert wieder lösbare Verbindung zwischen dem Grundkörper und dem ersten Teil der Pfanne hergestellt werden.

Vorteilhafterweise umfassen der erste Teil der Pfanne und der Grundkörper zusammenwirkende Führungsmittel. Dadurch können der Grundkörper und der erste Teil der Pfanne relativ zueinander positioniert werden, um auf besonders einfache Weise die Pfanne in dem Grundkörper definiert zur Erzielung einer gewünschten Inklination und/oder Anteversion zu verstellen.

Um die Inklination unabhängig von der Anteversion einzustellen, ist es vorteilhaft, verschiedene Pfannen und/oder Grundkörper vorzusehen, deren jeweilige zusammenwirkende Führungsmittel eine Variation der Kombination der unterschiedlichen Anteversions- und Inklinationswinkel ermöglichen.

In einer Weiterbildung der Erfindung sind zusätzlich oder alternativ zu den zusammenwirkenden Führungsmitteln formschlussbildende Mittel in der Pfanne und dem Grundkörper vorgesehen, die spezielle Rastpositionen umfassen, so dass stufenlos oder stufenweise vorbestimmte Inklinations- und Anteversionswinkel einstellbar sind. Durch das Vorhalten unterschiedlicher Pfannen und/oder Grundkörper lassen sich auch in dieser Ausführungsform Inklination und Anteversion teilweise unabhängig voneinander stufenlos oder in Stufenabschnitten einstellen. Dies erhöht die Variabilität des Einsatzes des modularen Hüftimplantats und stellt weiter eine besonders einfache und sichere Handhabung zur Einstellung der gewünschten Inklination und Anteversion bereit.

In einer Weiterbildung ist die Nut auf der Innenseite des Aufnahmebereichs des Grundkörpers und der Vorsprung auf der Außenseite der Pfanne angebracht. In diesem vorteilhaften Ausführungsbeispiel ist ein Einsetzen der Pfanne in den Grundkörper besonders einfach. Durch das Ineinandergreifen von Nut und Vorsprung an Grundkörper und Pfanne wird ein kraftaufnehmender formschlüssiger Verbund erreicht.

In einer Weiterbildung ist der Vorsprung ringförmig umlaufend ausgeformt. Damit ist eine stufenlose Einstellung von Inklinations- und Anteversionswinkel möglich. Es ist auch von Vorteil, wenn die Nut ringförmig umlaufend ausgeformt ist. Vorsprung und Nut können in anderen Ausführungsformen jedoch auch unterbrochen sein. In speziellen Ausführungsformen können der Vorsprung und die Nut sogar auf einzelne definiert angeordnete Pfosten bzw. Vertiefungen reduziert sein.

Es hat sich als zweckmäßig herausgestellt, insbesondere unter dem Aspekt der einfachen und zuverlässigen wie auch kostengünstigen Herstellung, wenn die Pfanne einen planen Rand aufweist, der vorzugsweise in einer Ebene liegt.

Durch die Ebene des planen Randes der Pfanne wird eine Flächennormale definiert. In gleicher Weise lässt sich durch den ringförmigen Vorsprung der Pfanne eine Fläche und somit eine zugehörige Flächennormale bestimmen. Die Normale der Ebene durch den Vorsprung und die Normale der Ebene des planen Randes weisen vorzugsweise einen Winkel α zwischen 0° und 30°, besonders vorzugsweise zwischen 5° und 20°, weiter vorzugsweise zwischen 15° und 20°, oder weiter vorzugsweise ca. 17° auf. Damit sind besonders günstige Anteversions- und Inklinationswerte erreichbar.

In entsprechender Weise bestimmen auch die Nut in dem Aufnahmebereich des Grundkörpers, bzw. die gemäss anderer Ausführungsformen entsprechenden Mittel, eine Ebene, durch die eine Flächennormale definiert ist. Der obere Rand des Aufnahmebereichs kann dabei ebenfalls eine Ebene und eine entsprechende Flächennormale bestimmen. In einer vorteilhaften Ausführungsform ist die Flächennormale der durch die Nut bestimmten Ebene um einen Winkel β geneigt zur Flächennormale der durch den oberen Rand des Aufnahmebereichs definierten Ebene, wobei dieser Neigungswinkel β vorzugsweise zwischen 0° und 30°, besonders vorzugsweise zwischen 5° und 25°, weiter vorzugsweise zwischen 10° und 22°, oder weiter vorzugsweise ca. 20° beträgt.

In einer vorteilhaften Kombination weist der Winkel β einen Wert im Bereich zwischen 0° und 30°, vorzugsweise im Bereich zwischen 5° und 20° , besonders bevorzugt im Bereich zwischen 10° und 20° oder weiter vorzugsweise ca. 17° auf, vorzugsweise in Kombination mit einem Winkel α im Bereich von 0° bis 30° , bevorzugt zwischen 5° und 25°, weiter vorzugsweise zwischen 10° und 22°, besonders bevorzugt von 20°.

In einem alternativen Ausführungsbeispiel ist der Grundkörper so ausgebildet, dass eine Ebene durch die Nut im Aufnahmebereich parallel zu einer durch den oberen Rand des Aufnahmebereichs des Grundkörpers definierten Ebene angeordnet ist.

Um das Einwachsverhalten zu verbessern, hat es sich in einem Ausführungsbeispiel als vorteilhaft herausgestellt, wenn die Pfanne und/oder der Grundkörper zumindest teilweise, vorzugsweise vollständig, aus Titan, einer Titanlegierung oder einer Titanverbindung bestehen. Alternativ könnte auch eine Edelstahl- oder Kobalt-Chrom-Legierung verwendet werden.

In einer Ausführungsform weist der Grundkörper zwei benachbarte radial abstehende Laschen und eine den Laschen gegenüberliegende Zunge auf. Dadurch lässt sich der Grundkörper besonders sicher und einfach an dem Beckenknochen befestigen.

In diesem Ausführungsbeispiel können die Laschen Bohrungen aufweisen, wodurch einfache Verschraubungen möglich sind.

Allgemein können in dem Hüftimplantat eine oder mehrere Bohrungen vorhanden sein, um eine Befestigung des Hüftimplantats am Beckenknochen und der einzelnen Komponenten des Hüftimplantats aneinander zu ermöglichen.

Geeigneterweise sind die Bohrungen zum Aufnehmen von Schraubverbindungen, die den Grundkörper und/oder die Pfanne mit dem Beckenknochen verbinden, geeignet.

Die Bohrungen können verschließbar sein, um das schädigende Eindringen von Abriebspartikeln in den Knochen zu verhindern oder den Kontakt von Knochenzement mit dem Knochen zu verhindern, wenn eine oder mehrere Bohrungen nicht zur Befestigung benötigt werden. Zum Verschließen der Öffnungen können Schrauben und/oder Stopfen verwendet werden. Die Stopfen können beispielsweise aus Polyethylen oder einem metallischen Implantatwerkstoff gefertigt sein.

Die Bohrungen könne mit einem Innengewinde zur Verwendung winkelstabiler Schrauben ausgestattet sein. Die Verwendung von etablierten Domschrauben hat sich in einem Ausführungsbeispiel als positiv herausgestellt.

Gemäß einem weiteren Beispiel weist ein modulares Hüftimplantat einen Grundkörper auf, der an einem Beckenknochen befestigbar ist und einen Aufnahmebereich mit einer zu einem Lagereinsatz der Hüftgelenksprothese komplementären Innenform aufweist. Der Aufnahmebereich weist eine Öffnung auf, die durch ein Verschlusselement verschließbar ist, wobei das Verschlusselement am Grundkörper befestigbar ist.

Durch die Öffnung wird auch nach dem Einsetzen des Hüftimplantats ein Zugang zum Beckenknochen ermöglicht. Durch das Verschlusselement wird eine Innenfläche, an der der Lagereinsatz der Hüftgelenksprothese befestigt werden kann, z.B. mit Zement, geschaffen.

Geeigneterweise weist das Verschlusselement auf einer dem Aufnahmebereich zugewandten Seite eine zu dem Lagereinsatz der Hüftgelenksprothese komplementäre Form auf. Dadurch kann ein guter Kontakt zwischen Lagereinsatz und Verschlusselement sichergestellt werden.

Vorteilhafterweise umfassen der Grundkörper und das Verschlusselement miteinander korrespondierende Befestigungsmittel, die beispielsweise ein Innengewinde im Grundkörper und ein mit diesem korrespondierendes Außengewinde im Verschlusselement umfassen können. Dadurch kann eine stabile und lange haltbare Verbindung zwischen Verschlusselement und Grundkörper geschaffen werden.

Geeigneterweise ist das Verschlusselement dafür ausgelegt, den Aufnahmebereich zu einer Vertiefung mit einer zu dem Lagereinsatz der Hüftgelenksprothese komplementären Innenform zu ergänzen. Dadurch kann eine große Kontaktfläche zwischen Lagereinsatz und Hüftimplantat geschaffen werden, wodurch eine gute Kraftübertragung zwischen Hüftimplantat und Hüftgelenksprothese sicher gestellt werden kann.

Der Aufnahmebereich kann als zweiseitig offene Kugelschale ausgebildet sein und das Verschlusselement kann als Kugelschale ausgebildet sein. Dadurch wird eine kugelschalenförmige Innenfläche des Hüftimplantats geschaffen, die zum Anbringen eines kugelförmigen Lagereinsatzes besonders geeignet ist.

Gemäß einem weiteren Beispiel umfasst ein Werkzeug zum Zusammenbau eines modularen Hüftimplantats, das einen Grundkörper, der an einem Beckenknochen befestigbar ist, und ein Bauteil, das dafür ausgebildet ist, mit einem Gewinde am Grundkörper verschraubt zu werden, umfasst, wobei im Grundkörper mindestens eine Bohrung ausgebildet ist, die eine Achse aufweist, die parallel zu einer Achse des Gewindes am Grundkörper ist, einen Führungsstab, der in die Bohrung im Grundkörper einführbar ist, eine Führungshülse, die entlang des Führungsstabs verschiebbar ist und ein Eindrehinstrument, das mit der Führungshülse verbunden ist. Eine Achse des Eindrehinstruments ist parallel zum Führungsstab.

Mit Hilfe dieses Werkzeugs kann das Einschrauben des Bauteils in den Grundkörper einfacher durchgeführt werden. Durch den Führungsstab kann das Einschraubwerkzeug und damit auch das Gewinde am Bauteil im wesentlichen parallel zum Gewinde im Grundkörper ausgerichtet werden. So kann ein Verkanten des Bauteils vermieden werden. Dadurch können Fehler bei der Implantation des Hüftimplantats, die zu einer verringerten Stabilität des Hüftimplantats führen könnten, vermieden werden.

Der Führungsstab kann an einem Ende ein Außengewinde aufweisen, das dafür ausgebildet ist, mit einem Innengewinde in der Bohrung des Grundkörpers verschraubt zu werden. Dadurch kann der Führungsstab während der Benutzung des Werkzeugs stabil am Grundkörper gesichert werden.

Geeigneterweise ist das Eindrehinstrument um den Führungsstab herum schwenkbar. Dadurch kann das Eindrehinstrument nach dem Einsetzen des Führungsstabs in eine zum Einschrauben des Bauteils geeignete Position gebracht werden, wobei die parallele Ausrichtung der Achse des Einschraubwerkzeugs zum Führungsstab erhalten bleibt.

Vorteilhafterweise umfasst das Eindrehinstrument eine Einrichtung zum lösbaren Befestigen des Bauteils des Hüftimplantats am Eindrehinstrument. So kann das Bauteil mit Hilfe des Eindrehinstruments in einer zum Einschrauben in das Gewinde des Grundkörpers geeigneten Ausrichtung an den Grundkörper herangeführt werden.

Die Erfindung wird nachfolgend unter Bezugnahme auf die Zeichnung näher erläutert. Es zeigen:
- Fig. 1: das erfindungsgemäße modulare Hüftimplantat, im in den Beckenknochen eingebauten Zustand in einer perspektivischen schematischen Ansicht,
- Fig. 2: eine perspektivische schematische isolierte Ansicht des erfindungsgemäßen modularen Hüftimplantates mit in den Grundkörper eingeschraubter Pfanne,
- Fig. 3: eine perspektivische isolierte Darstellung des Grundkörpers aus den Figuren 1 und 2 mit einer kranialen Domschraube, und einer kaudal eingebrachten Schraube.
- Fig. 4: eine schematische Schnittansicht durch die Pfanne und den Grundkörper aus den Figuren 1 bis 3, wobei die Pfanne einen Vorsprung und der Aufnahmebereich des Grundkörpers eine Nut aufweist,
- Fig. 5: eine Detailansicht des Bereichs V aus Fig. 4,
- Fig. 6: eine alternative Ausführungsform der Erfindung in schematischer Schnittansicht durch die Pfanne und den Grundkörper, wobei die Pfanne eine Nut und der Grundkörper einen Vorsprung aufweist,
- Fig. 7: eine Detailansicht des Bereichs VII aus Fig. 6 und
- Fig. 8a: eine schematische Perspektivansicht eines Hüftimplantats gemäß einer weiteren Ausführungsform der vorliegenden Erfindung;
- Fig. 8b: ein schematisches Schnittbild der in Fig. 8a gezeigten Ausführungsform;
- Fig. 9a bis 9d: schematische Perspektivansichten der in Fig. 8a und Fig. 8b gezeigten Ausführungsform in vier verschiedenen Stadien des Zusammenbaus;
- Fig. 10: ein schematisches Schnittbild durch die in den Fig. 8a bis 9d gezeigte Ausführungsform in zusammengebautem Zustand;
- Fig. 11 a: eine schematische Perspektivansicht eines Hüftimplantats gemäß einer weiteren Ausführungsform der vorliegenden Erfindung;
- Fig. 11b eine: schematische Perspektivansicht des in Fig. 11a gezeigten Hüftimplantats in einem Stadium des Zusammenbaus; und
- Fig. 12: eine schematische Perspektivansicht eines Hüftimplantats gemäß noch einer weiteren Ausführungsform der vorliegenden Erfindung.

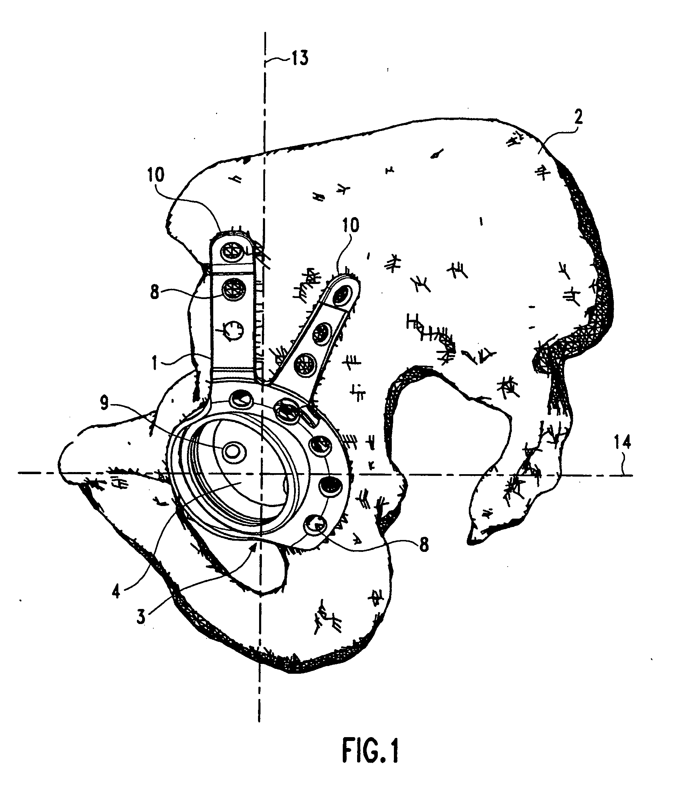

Figur 1 zeigt ein erfindungsgemäßes modulares Hüftimplantat mit einem Grundkörper 1, der an einem Beckenknochen 2 befestigt ist. Der Grundkörper 1 weist einen schalenförmigen Aufnahmebereich 3 auf. In den Aufnahmebereich 3 ist eine Pfanne 4 eingesetzt.

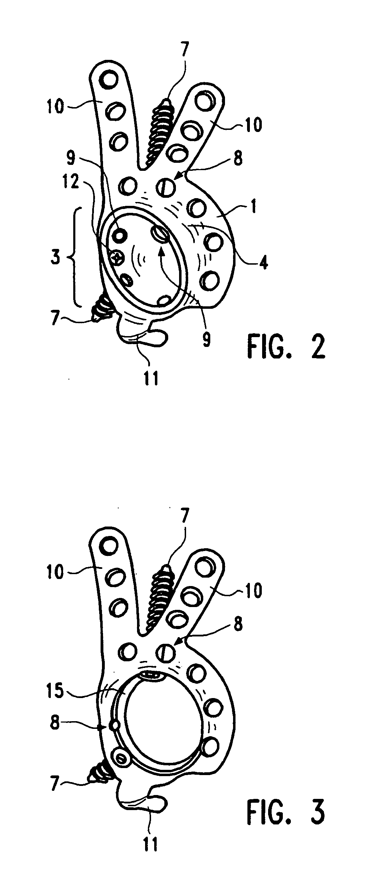

Der Grundkörper 1 weist Befestigungsmittel zur Befestigung des Grundkörpers 1 am Beckenknochen auf. Als Befestigungsmittel finden Schrauben, wie etwa eine Domschraube 7, Verwendung. Die Befestigungsmittel, insbesondere die Domschraube 7, sind in Figur 1 nicht dargestellt. Domschrauben 7 sind jedoch in den Figuren 2 und 3 dargestellt. In den Grundkörper 1 sind Bohrungen 8 eingearbeitet. Weitere Bohrungen 9 sind auch in die Pfanne 4 eingearbeitet.

Der Grundkörper 1 weist zwei Laschen 10 und eine Zunge 11 auf. Diese ist in Fig. 1 nicht sichtbar, aber in den Fig. 2 und 3 dargestellt. Die Laschen 10 sind zueinander benachbart angeordnet. Sie befinden sich auf der gegenüberliegenden Seite der Zunge 11. Die Laschen 10 sind ebenfalls mit Bohrungen 8 versehen, wie auch der Aufnahmebereich 3. Die Zunge 11, die beispielweise bohrungsfrei ausgebildet ist, ist hakenförmig zum Umgriff um einen Teil des Beckenknochens 2 ausgebildet. Zur Fixierung des Grundkörpers 1 am Beckenknochen 2 können zusätzlich zu der Domschraube 7 durch die Bohrungen 9 in den Laschen 10 Schrauben in den Beckenknochen 2 eingeschraubt sein.

Die Bohrungen 8 und/oder 9 können auch mit einem Innengewinde ausgeführt sein, um die Einbringung winkelstabiler Schrauben zu ermöglichen.

Der Aufnahmebereich 3 des Grundkörpers 1 ist konkav ausgestaltet und hat die Form einer ringförmigen, nach unten, d.h. gegen den Beckenknochen, offenen Schale. In diesen ringförmigen teilschalenförmigen konkaven Bereich des Aufnahmebereichs des Grundkörpers ist die mit korrespondierender konvexer Außenfläche gestaltete Pfanne 4 einsetzbar und kommt dort zur Anlage. Der Aufnahmebereich 3 und die konvexe Außenfläche der Pfanne können jeweils einen im wesentlichen kugelförmigen Oberflächenbereich aufweisen, deren Radien derart ausgelegt sind, dass die Oberflächenbereiche des Aufnahmebereichs und der Pfanne flächig aufeinander aufliegen.

Zur Befestigung der Pfanne 4 in dem Grundkörper 1 sind Schrauben 12 durch die Bohrungen 8 im Grundkörper und in der Pfanne geführt. Die Bohrungen 8 im Grundkörper 1 weisen dabei vorzugsweise ein Innengewinde auf, in welches eine Verbindungsschraube 12, wie etwa in Figur 2 dargestellt, eingreift. Durch selektives Influchtbringen der Bohrungen 8 und 9 miteinander, lassen sich verschiedene Winkelstellungen der Pfanne 4 in Bezug auf den Grundkörper 1 realisieren. Die dann durch die Bohrungen 8 und 9 durchgreifende Schraubverbindung fixiert dauerhaft die Pfanne im Grundkörper.

In die halbkugelschalenförmig ausgebildete Pfanne 4 greift eine Hüftgelenkprothese (nicht dargestellt), nämlich ein im wesentlichen kugelförmiges Ende eines künstlichen Hüftgelenks, insbesondere eine künstliche Gelenkpfanne, die durch einen Lagereinsatz gebildet wird, ein. Der Lagereinsatz kann einen Kunststoff, beispielsweise Polyethylen, umfassen. In speziellen Ausführungsformen der vorliegenden Erfindung kann UHMW (Ultra High Molecular Weight)-PE oder crosslinked-PE verwendet werden. Alternativ kann der Lagereinsatz ein Keramikmaterial, z.B. Aluminiumoxyd- oder Zirkonoxydkeramik oder eine Metall-Legierung, z. B. CoCr28Mo6 umfassen.

Die Pfanne 4 lässt sich in manchen Ausführungsformen in dem Aufnahmebereich des Grundkörpers um drei zueinander orthogonale Achsen bewegen. Diese Achsen sind wie folgt definiert: Eine erste Horizontalachse (nicht gezeigt) erstreckt sich durch die zwei Drehpunkte der beiden Hüftgelenke des Beckenknochens 2. Eine zweite Horizontalachse 14 steht senkrecht auf der ersten Horizontalachse und einer Vertikalachse 13. Durch Drehung der Pfanne um die Vertikalachse 13 lässt sich die Anteversion einstellen. Durch Drehung um die Horizontalachse 14 lässt sich die Inklination einstellen.

In Figur 2 ist die mittels der Verbindungsschraube 12 gesicherte Pfanne 4 im Grundkörper 1, und zwar im Aufnahmebereich 3, dargestellt. Zwei Domschrauben 7, oder andere geeignete Schrauben, ermöglichen die Fixierung von Grundkörper 1 mit dem Beckenknochen 2 (nicht dargestellt). Die Anordnung der Bohrungen 8 und 9, in Grundkörper 1 und Pfanne 4 lässt sich variieren. Je nach Anordnung der Bohrungen 8 und 9, insbesondere relativ zueinander, lassen sich unterschiedliche Winkelstellungen zur Erzielung verschiedener Einstellungen der Inklination und Anteversion erreichen.

Die näher am kaudalen Haken liegende Schraube 7 kann in dem im Knochenkontakt stehenden Bereich auch als glatter Zapfen ausgebildet sein.

In Figur 3 ist ein singulärer Grundkörper 1 mit zwei Domschrauben 7 dargestellt. Im ringförmigen Aufnahmebereich 3, der in sich konkav gewölbt ist, ist eine Nut 15 eingearbeitet, die grundkörperseitig ein Mittel zur Führung und zur Einstellung der Position der eingesetzten Pfanne bildet. Die Nut 15 erstreckt sich ringförmig durch den gesamten Aufnahmebereich 3. Es ist auch möglich, die Nut 15 unterbrochen auszugestalten.

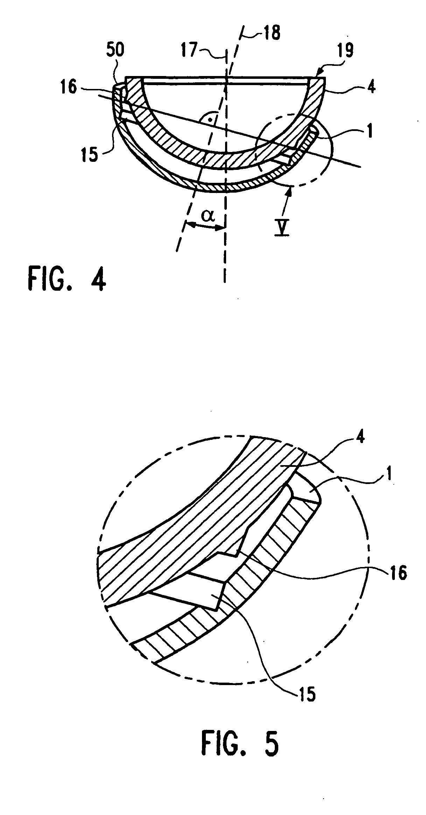

In Figur 4 ist das Eingreifen eines pfannenseitig ausgebildeten Mittels zur Führung und zur Einstellung der Position der eingesetzten Pfanne in Form eines Vorsprungs 16, der auf der Außenseite der Pfanne 4 vorhanden ist, in die Nut 15 des Grundkörpers 1 dargestellt. Ein Formschluss zwischen Nut 15 und Vorsprung 16 ist im eingesetzten Zustand der Pfanne 4 in den Grundkörper 1 die Folge. Dabei werden die wirkenden Kräfte über die Nut 15 an den Vorsprung 16 weitergegeben. Der Vorsprung 16 läuft beispielsweise ringförmig um die gesamte Außenfläche der Pfanne 4. Eine oder mehrere Unterbrechungen des Vorsprungs sind alternativ möglich. Der Vorsprung 16 und die Nut 15 bilden zusammenwirkende Führungsmittel.

Eine Normale 18 durch eine Ebene, in der der Vorsprung 16 liegt, ist im in der Figur 4 dargestellten Ausführungsbeispiel nicht parallel zu einer Normalen 17 der Ebene, in der ein oberer Rand 19 der Pfanne liegt. Die Normalen 17 und 18 haben beispielsweise einen Winkel von ca. 17° zwischen einander aufgespannt. In Bezug auf einen oberen Rand 50 des Grundkörpers ist ebenfalls eine Ebene definiert. Bezüglich dieser Ebene des Randes 50 des Grundkörpers weist die Ebene der Nut in dem Grundkörper einen Winkel von ca. 20° auf. Durch Verdrehen der Pfanne 4 in dem Aufnahmebereich des Grundkörpers lassen sich kontinuierlich Winkel zwischen der Ebene des Randes der Pfanne und des Grundkörpers im Bereich von 3° bis 37° einstellen.

In Figur 5 ist im größeren Detail der Vorsprung 16 und die Nut 15 dargestellt. In den Figuren 4 und 5 sind die Fixiermittel noch nicht miteinander in Eingriff, da die Pfanne 4 noch nicht vollständig in den Grundkörper 1 eingesetzt ist. Im komplett eingesetzten Zustand befindet sich der Vorsprung 16 in der Nut 15.

Der Winkel der Normalen durch die Ebene des an der Pfanne befindlichen Führungsmittels ist als Winkel α in den Figuren gekennzeichnet und wird zwischen der Normalen 17 durch den Rand der Pfanne 4 und der Normalen 18 durch die Ebene des Führungsmittels der Pfanne 4 gemessen.

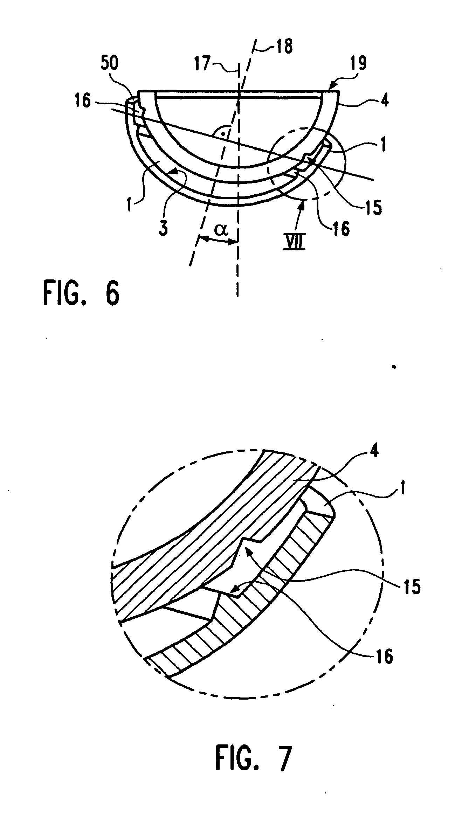

Die Figuren 6 und 7 zeigen eine alternative Ausgestaltung des modularen Hüftimplantats. Der Vorsprung 16 ist dabei im Grundkörper 1 ausgeformt und die Nut 15 ist dabei auf der Außenseite der Pfanne 4 ausgeformt.

Der Rand 19 kann auch eine Überhöhung, beispielsweise von ca. 10°, aufweisen, der dann nicht in derselben Ebene wie der Rest des Randes 19 liegt.

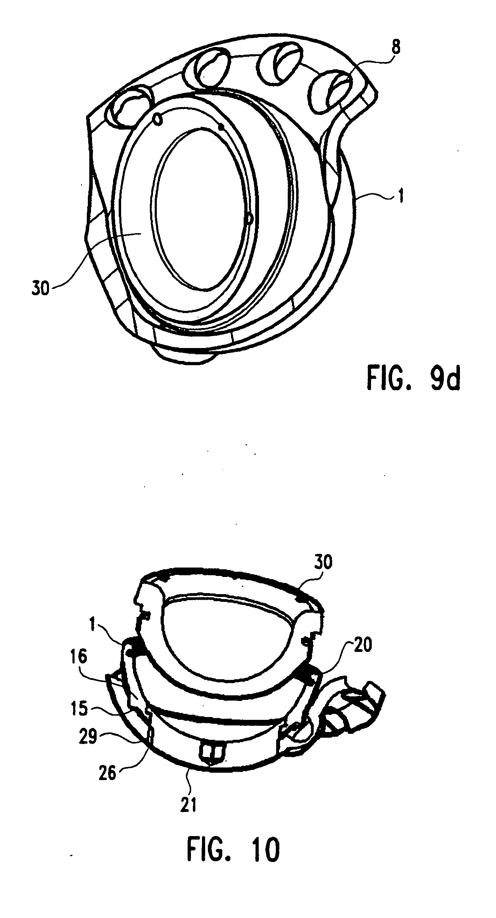

Gegenüber einem rotationssymmetrisch ausgestalteten Lagereinsatz kann auch der Rand des Lagereinsatzes 30 (Figur 10) eine Überhöhung im Bereich von 10° bis 20° aufweisen.

Durch Drehung der Pfanne in dem Grundkörper lassen sich die Werte eines Winkels δ der Flächennormalen durch den Pfannenrand 19 und durch den Rand 50 des Aufnahmebereichs von 0° bis 34° einstellen, wenn z.B. die in dem Aufnahmebereich des Grundkörpers vorgesehene ringförmige Nut ebenfalls um einen Winkel von 17° gegen die durch den oberen Rand des Aufnahmebereichs gebildete Fläche angestellt ist. Andere Einstellbereiche für den Winkel δ sind möglich, abhängig von den jeweiligen Anstellwinkeln α und β sowie von den durch Nut und Vorsprung jeweils bestimmten Ebenen.

Anstelle von Mitteln, bei denen sich durch Drehen der Pfanne der δ-Wert und damit die Inklinations- und Anteversionswinkel einstellen lassen, können auch spezielle Rastpositionen in Pfanne 4 und Grundkörper 1 vorgesehen sein, wobei Mittel zum Hervorrufen eines Formschlusses, also entsprechend der Nut 15 und des Vorsprungs 16, so angeordnet sind, dass sich stufenweise die individuell benötigten Werte einstellen lassen.

Durch das Vorhalten unterschiedlicher Pfannen für einen Grundkörper lassen sich auch Anteversionswerte unabhängig von dem Inklinationswert einstellen. Es ist auch möglich, mehrere Nuten oder Vorsprünge in Pfanne 4 oder Grundkörper 1 vorzusehen. Auch ist es möglich, sowohl Vorsprünge als auch Nuten, jeweils an Pfanne 4 und/oder am Grundkörper 1 vorzusehen.

Die Nuten und Vorsprünge können dabei auch abschnittsweise ausgebildet sein. In speziellen Ausführungsformen der vorliegenden Erfindung können die Nuten und Vorsprünge auch auf einzelne definiert angeordnete Pfosten und Vertiefungen reduziert sein.

Die Pfanne und der Grundkörper sind zumindest teilweise, vorzugsweise vollständig, aus Titan oder einer Titanlegierung oder -verbindung hergestellt. Als Titanlegierung ist TiAl6V4 besonders geeignet. Ein Grundkörper aus Reintitan ermöglicht eine besonders einfache Anpassung des Hakens und/oder der Laschen an die individuelle Anatomie des Beckenknochens des Patienten, da dieses Material aufgrund seiner Duktilität auch bei Raumtemperatur gut verformbar ist.

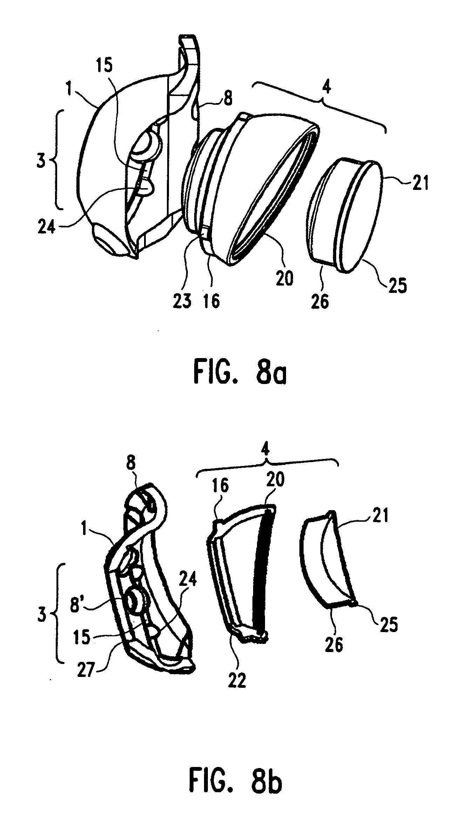

Weitere Ausführungsformen der vorliegenden Erfindung werden mit Bezug auf die Fig. 8a bis 10 beschrieben.

Fig. 8a zeigt eine schematische Perspektivansicht eines modularen Hüftimplantats. Fig. 8b zeigt ein schematisches Schnittbild des in Fig. 8a gezeigten Hüftimplantats. Dieses umfasst einen Grundkörper 1 und eine Pfanne 4, die einen ersten Teil 20 und einen zweiten Teil 21 umfasst. Anders als in den mit Bezug auf die Fig. 1 bis 7 beschriebenen Ausführungsformen sind keine Zunge 11 und keine Laschen 10 vorgesehen. In anderen Ausführungsformen kann der Grundkörper 1 jedoch Laschen und eine Zunge aufweisen.

Der Grundkörper 1 ist an einem Beckenknochen befestigbar. Zu diesem Zweck können Schrauben durch Bohrungen 8 im Grundkörper geführt und mit dem Beckenknochen verschraubt werden. Die Bohrungen 8 können am Umfang des Aufnahmebereichs 3 oder im Inneren des Aufnahmebereichs 3 angeordnet sein In Ausführungsformen, in denen der Grundkörper 1 Laschen aufweist, könne die Bohrungen auch in den Laschen vorgesehen sein. Mindestens eine Bohrung 8' im Inneren des Aufnahmebereichs 3, die in der Ansicht der Fig. 8a durch die Pfanne 4 verdeckt ist, ist in Fig. 8b gezeigt.

Wie in den oben mit Bezug auf die Fig. 1 bis 7 beschriebenen Ausführungsformen können die Bohrungen ein Innengewinde umfassen, so dass die Schrauben winkelstabil angebracht werden können. Insbesondere können zur Verbindung des Grundkörpers 1 mit dem Beckenknochen Domschrauben verwendet werden.

In anderen Ausführungsformen kann der Grundkörper auch durch eine Zementschicht mit dem Beckenknochen verbunden werden, wobei ein den Fachleuten bekannter Knochenzement verwendet werden kann. In solchen Ausführungsformen können die Bohrungen 8, 8' des Grundkörpers weggelassen oder verschlossen werden. Alternativ können eine Befestigung durch Zement und eine Befestigung durch Schrauben gleichzeitig verwendet werden, wodurch eine besonders stabile Befestigung erzielt werden kann.

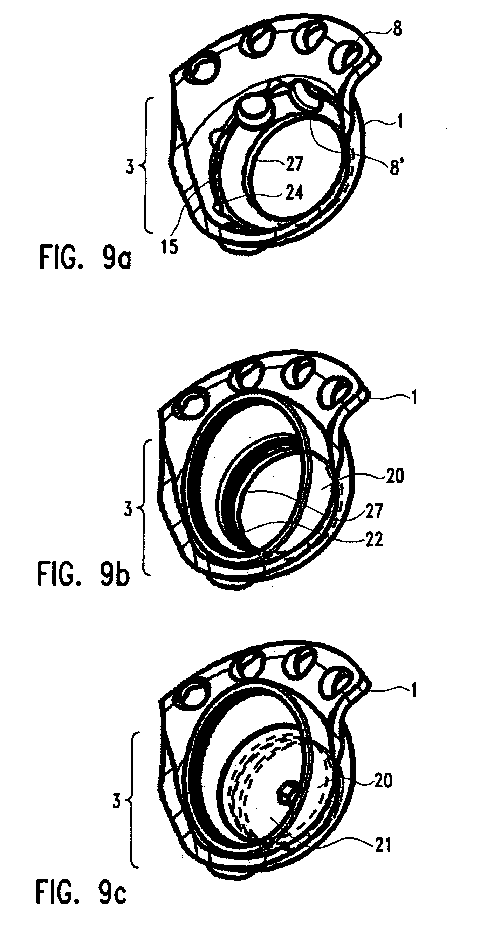

Der Grundkörper 1 umfasst einen Aufnahmebereich 3, in den die Pfanne 4 eingesetzt werden kann. Die Pfanne 4 weist eine zu dem Aufnahmebereich 3 komplementäre Außenform auf. In der in den Fig. 9 bis 10 gezeigten Ausführungsform ist der Aufnahmebereich 3 als eine zweiseitig offene Kugelschale ausgebildet. Im Inneren des Aufnahmebereichs 3 ist dabei eine Öffnung 27 vorgesehen. Wenn das Hüftimplantat zusammengesetzt ist, liegt die in Fig. 8a und 8b dem Grundkörper 1 zugewandte Seite des ersten Teils 20 der Pfanne 4 auf dem Aufnahmebereich 3 auf (vgl. Fig. 9c). Der erste Teil 20 der Pfanne 4 ist dabei als eine zweiseitig offene Kugelschale ausgebildet, wobei die Radien des Aufnahmebereichs 3 und des zweiten Teils 21 der Pfanne 4 derart aufeinander abgestimmt sind, dass ein flächiger Kontakt zwischen dem Aufnahmebereich 3 und der Pfanne 4 entsteht. Dadurch werden mechanische Kräfte von der Pfanne 4 gleichmäßig auf den Grundkörper 1 übertragen.

Der erste Teil 20 der Pfanne 4 weist eine Öffnung 22 auf. Wenn der erste Teil 20 der Pfanne 4 in den Grundkörper 1 eingesetzt ist, liegen die Öffnungen 22, 27 des ersten Teils 20 und des Grundkörpers 1 einander gegenüber, so dass die Öffnung 27 des Grundkörpers zugänglich ist. Die Öffnungen 22, 27 können eine im wesentlichen kreisförmige Gestalt haben. Die Öffnung 22 kann einen Durchmesser aufweisen, der mindestens so groß ist wie der Durchmesser der Öffnung 27, so dass die gesamte Öffnung 27 auch dann freiliegt, wenn der erste Teil 20 der Pfanne 4 in den Grundkörper 4 eingesetzt ist.

Der zweite Teil 21 der Pfanne 4 weist eine Form auf, die mit der Öffnung 22 im ersten Teil 20 korrespondiert. Im Fall einer im wesentlichen kreisförmigen Öffnung 22 hat der zweite Teil 21 der Pfanne 4 eine runde Form. Beispielsweise kann er als Kugelschale ausgebildet sein.

Der zweite Teil 21 der Pfanne 4 ist am Grundkörper 1 befestigbar. Zu diesem Zweck können der zweite Teil 21 der Pfanne 4 und der Grundkörper 1 miteinander korrespondierende Befestigungsmittel umfassen. In der in Fig. 8a bis 10 gezeigten Ausführungsform umfasst der Grundkörper 1 ein Innengewinde 29, das am Umfang der Öffnung 27 ausgebildet ist. Der zweite Teil 21 der Pfanne 4 weist an seinem Umfang ein Außengewinde 26 auf, das zum Innengewinde des Grundkörpers 1 passt (vgl. Fig. 10). Das Außengewinde 26 kann selbstsichernd ausgebildet sein.

Auf der in der Perspektive der Fig. 8a und 8b nach rechts zeigenden Seite weist der zweite Teil 21 der Pfanne 4 einen Vorsprung 25 auf. Ein Durchmesser des äußeren Umfangs des Vorsprungs 21 ist größer als der Durchmesser der Öffnung 22 im ersten Teil der Pfanne 4, so dass der zweite Teil 21 der Pfanne 4 nicht vollständig durch die Öffnung 22 hindurchtreten kann. Der Rest des zweiten Teils 21 weist dagegen einen kleineren äußeren Durchmesser auf als die Öffnung 22, so dass der zweite Teil der Pfanne 4 durch die Öffnung 22 geführt werden kann.

Bei der Implantation des Hüftimplantats kann zunächst der Grundkörper 1 mit Hilfe von Schrauben im Beckenknochen des Patienten fixiert werden. Die Schrauben und der Beckenknochen sind in Fig. 9a nicht gezeigt. Anschließend wird, wie in Fig. 9b dargestellt, zunächst der erste Teil 20 der Pfanne in den Grundkörper 1 eingesetzt und zwar so, dass sich die Öffnungen 22 und 27 gegenüber liegen. Durch Drehen des ersten Teils 20 relativ zum Grundkörper 1 können Inklination und Anteversion eingestellt werden. Zu diesem Zweck kann, wie in den oben mit Bezug auf Fig. 1 bis 7 beschriebenen Ausführungsformen, eine Ebene, in der der obere Rand des ersten Teils 20 der Pfanne 4 liegt, gegenüber einem oberen Rand des Grundkörpers geneigt sein. Ferner können am ersten Teil 20 der Pfanne 4 und am Grundkörper zusammenwirkende Führungsmittel ähnlich den in den mit Bezug auf Fig. 1 bis 7 beschriebenen Ausführungsformen an der Pfanne 4 und am Grundkörper 1 vorgesehenen Führungsmitteln vorgesehen sein. Beispielsweise umfasst der erste Teil 20 der Pfanne 4 einen Vorsprung 16 und der Grundkörper 1 eine mit diesem korrespondierende Nut 15. Durch Ausbuchtungen 23 des Vorsprungs 16 und Vertiefungen 24 der Nut 15 werden Rastpositionen des ersten Teils der Schale 20 mit vorbestimmter Inklination und Anteversion definiert.

In anderen Ausführungsformen der vorliegenden Erfindung können die Rastpositionen durch andere Merkmale des Grundkörpers 1 und des ersten Teils 20 der Pfanne 4 definiert sein. In solchen Ausführungsformen können im Vorsprung 16 Vertiefungen vorgesehen sein und die Nut 15 kann an einigen Stellen unterbrochen sein. In weiteren Ausführungsformen können die Merkmale des Grundkörpers 1 und des ersten Teils 20 der Pfanne 4, die die Rastpositionen definieren, an anderen Stellen als an der Nut 15 und dem Vorsprung 16 vorgesehen sein, z.B. neben der Nut 15 bzw. dem Vorsprung 16.

Anschließend wird, wie in Fig. 9c gezeigt, der zweite Teil 21 der Pfanne 4 durch die Öffnung 27 im ersten Teil geführt und das Außengewinde 26 des zweiten Teils wird mit dem Innengewinde 27 des Grundkörpers 1 verschraubt. Der Vorsprung 25 des zweiten Teils liegt dann auf dem ersten Teil 20 der Pfanne 4 auf, wodurch der erste Teil 20 relativ zum Grundkörper 1 fixiert wird.

Der zweite Teil 21 der Pfanne 4 kann Bohrungen 9 aufweisen, die zum Aufnehmen von Schrauben, die mit dem Beckenknochen verbindbar sind, ausgebildet sind. So kann der zweite Teil 21 unabhängig vom Grundkörper 1 am Beckenknochen befestigt werden, wodurch ein besonders stabiler Halt des zweiten Teils 21 der Pfanne 4 erreicht werden kann.

Daraufhin kann, wie in den Fig. 9d und 10 gezeigt, ein Lagereinsatz 30 in der Pfanne 4 befestigt werden, was mit Hilfe von dem Fachmann bekannten Mitteln geschehen kann.

In weiteren Ausführungsformen kann alternativ oder zusätzlich zur Befestigung des zweiten Teils 21 der Pfanne 4 mit Hilfe des Gewindes 26 eine Befestigung durch eine Zementschicht vorgesehen sein.

Die Befestigung des ersten Teils 20 der Pfanne 4 am Grundkörper muss nicht, wie in den oben mit Bezug auf die Fig. 8a bis 10 beschriebenen Ausführungsformen, mit Hilfe des Vorsprungs 25 des zweiten Teils 21 der Pfanne 4 geschehen. In weiteren Ausführungsformen kann der erste Teil 20 der Pfanne 4 dafür ausgelegt sein, mit Hilfe einer Zementschicht am Grundkörper 1 und dem zweiten Teil 21 der Pfanne 4 befestigt zu werden.

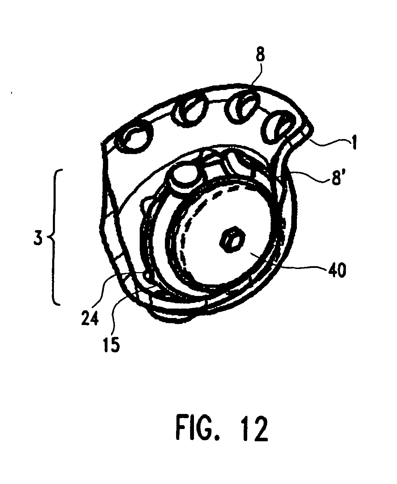

Ein Hüftimplantat gemäß der vorliegenden Erfindung muss keine Pfanne 4 aufweisen. In weiteren Ausführungsformen der Erfindung, die im Folgenden mit Bezug auf Fig. 12 beschrieben werden, kann der Lagereinsatz 30 einer Hüftgelenksprothese durch eine Zementschicht im Aufnahmebereich 3 des Grundkörpers 1 befestigt werden.

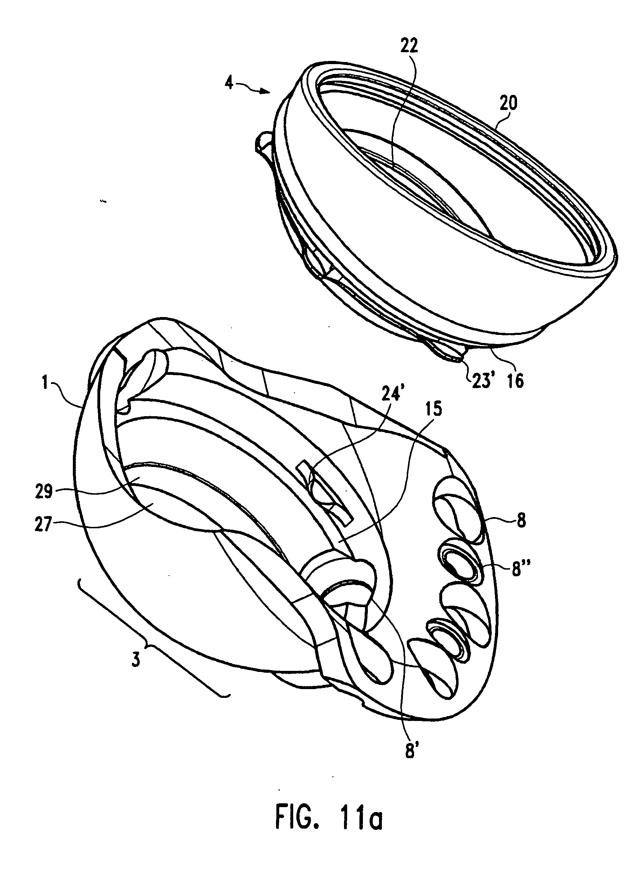

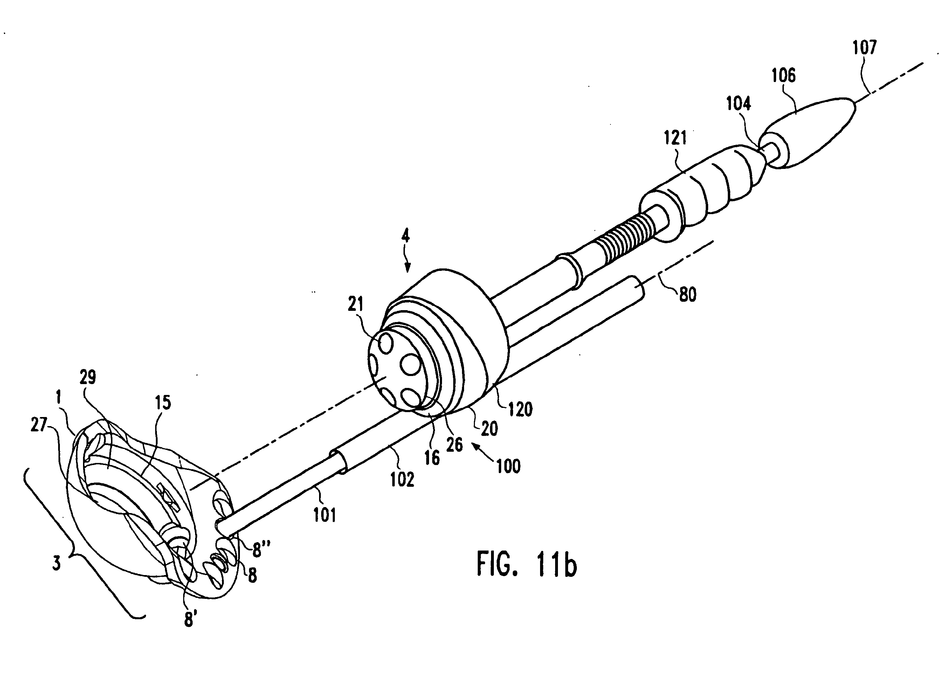

Fig. 11 a zeigt eine schematische Explosionszeichnung eines Hüftimplantats gemäß einer weiteren Ausführungsform der vorliegenden Erfindung. Eine schematische Perspektivansicht des Hüftimplantats in einem Stadium des Zusammenbaus ist in Fig. 11b gezeigt. Das Hüftimplantat umfasst einen Grundkörper 1 und eine Pfanne 4, die einen ersten Teil 20 und einen zweiten Teil 21 (nur in Fig. 11b gezeigt) umfasst. Der Grundkörper 1 umfasst einen Aufnahmebereich 3 für die Pfanne 4 und Bohrungen 8, 8', die mit einem Innengewinde zur Aufnahme von Schrauben ausgestattet sein können. Im Grundkörper 1 und im ersten Teil 20 der Pfanne 4 sind Führungsmittel in Form einer Nut 15 und eines Vorsprungs 16 vorgesehen. Am zweiten Teil 21 der Pfanne 4 ist ein Vorsprung angebracht, der größer als der Durchmesser der Öffnung 22 des ersten Teils 20 der Pfanne 4 ist, um ein Durchrutschen des zweiten Teils 21 durch die Öffnung 22 zu verhindern. Diese Merkmale können ähnlich den entsprechenden Merkmalen in den oben mit Bezug auf die Fig. 8a bis 10 beschriebenen Ausführungsformen ausgebildet sein, wobei für entsprechende Merkmale dieselben Bezugszeichen verwendet werden.

In der Öffnung 27 des Grundkörpers 1 kann ein Innengewinde 29 vorgesehen sein und am zweiten Teil 21 der Pfanne kann ein mit dem Innengewinde 29 korrespondierendes Außengewinde 26 vorgesehen ein. Dadurch kann der zweite Teil 21 der Pfanne 4 mit dem Grundkörper 1 verschraubt werden, um den ersten Teil 20 und den zweiten Teil 21 am Grundkörper 1 zu fixieren. Das Innengewinde 29 und das Außengewinde 26 können als Feingewinde ausgebildet sein. In einer speziellen Ausführungsform können das Innengewinde 29 und das Außengewinde 26 eine Ganghöhe (Gewindesteigung) im Bereich von 0,5 bis 2 aufweisen. Durch das Feingewinde kann die Stabilität der Verbindung zwischen dem Grundkörper 1 und der Pfanne 4 verbessert werden. Insbesondere kann durch das Feingewinde bei einer Dicke des Grundkörpers im Bereich der Öffnung 27 von ungefähr 3 mm eine ausreichend stabile Verbindung geschaffen werden, die sich auch bei Wechsellasten und minimalen Verformungen des Hüftimplantats nicht löst.

Der erste Teil 20 der Pfanne 4 ist in mehreren Positionen relativ zum Grundkörper 1 fixierbar. Zu diesem Zweck sind am Grundkörper 1 Vorsprünge 24' angebracht, wobei in der Perspektive der Fig. 11 a nur ein Vorsprung 24' zu sehen ist. Am ersten Teil 20 der Pfanne 4 befinden sich mehrere Vorsprünge 23'. Zum Vorverriegeln des ersten Teils 20 am Grundkörper 1 kann jeweils einer der Vorsprünge 23' neben den Vorsprüngen 24' eingesetzt werden. Anschließend kann der erste Teil 20 der Pfanne 4 gedreht werden, so dass jeweils ein Vorsprung 23' unter einen Vorsprung 24' zu liegen kommt.

Die Vorsprünge 23' und 24' bilden einen Bajonettverschluss, so dass ein Herausfallen des ersten Teils 20 der Pfanne 4 aus dem Grundkörper 1 vermieden werden kann. Die Vorsprünge 23' am ersten Teil 20 der Pfanne 4 und die Vorsprünge 24' im Grundkörper 1 sind symmetrisch angeordnet, so dass der erste Teil 20 in mehreren verschiedenen Positionen am Grundkörper 1 angebracht werden kann, um die Anteversion und die Inklination des Hüftimplantats an die Anatomie des Patienten anzupassen.

Fig. 11b zeigt eine schematische Perspektivansicht des Hüftimplantats in einem Stadium des Zusammenbaus.

Zum Vorverriegeln des ersten Teils 20 der Pfanne 4 und zum Einschrauben des zweiten Teils 21 der Pfanne 4 im Grundkörper 1 kann ein Werkzeug 100 verwendet werden. Das Werkzeug 100 umfasst einen Führungsstab 101. Der Führungsstab 101 kann an einem Ende ein Außengewinde umfassen. Ein zu diesem Außengewinde korrespondierendes Innengewinde ist in einer Bohrung 8" im Grundkörper 1 vorgesehen. Eine Achse 80 der Bohrung 8" ist zu einer Achse 107 des Innengewindes 29 im Grundkörper im wesentlichen parallel. Wenn der Führungsstab 101 in die Bohrung 8" eingeschraubt wird, ist der Führungsstab 101 somit im wesentlichen parallel zur Achse 107 des Innengewindes 29.

Das Werkzeug 100 umfasst ferner eine Führungshülse 102, die den Führungsstab 101 umschließt und entlang des Führungsstabs 101 verschiebbar ist. An der Führungshülse 102 ist durch eine Halterung (in der Perspektive der Fig. 11b nicht sichtbar) ein Eindrehinstrument 104 angebracht, das um eine Achse drehbar ist, die parallel zur Achse des Führungsstabs 101 ist. Das Eindrehinstrument 104 kann beispielsweise als Schraubendreher ausgebildet sein, und einen Griff 106 sowie eine Klinge umfassen, wobei die Klinge dafür ausgebildet ist, in einen am zweiten Teil 21 der Pfanne 4 angebrachten Schraubenkopf einzugreifen. Beispielsweise kann der zweite Teil 21 der Pfanne einen Innensechskant-Schraubenkopf aufweisen.

Weiterhin umfasst das Werkzeug 100 einen Adapter 120, der lösbar mit dem ersten Teil 20 der Pfanne 4 verbindbar ist, beispielsweise durch einen Klickmechanismus. Der Adapter 120 kann mit einem Griff 121 verbunden sein, der eine Längsbohrung aufweisen kann, durch die das Eindrehinstrument 104 geführt ist, wobei das Eindrehinstrument 104 und der zweite Teil 21 der Pfanne 4 relativ zum Adapter 120 und zum ersten Teil 20 drehbar sind.

Ein Abstand zwischen dem Eindrehwerkzeug 104 und dem Führungsstab 101 ist im wesentlichen gleich einem Abstand zwischen der Achse 80 der Bohrung 8" und der Achse 107 des Innengewindes 29. Wenn der Führungsstab 101 in die Bohrung 8" eingesetzt ist, kann das Eindrehwerkzeug 104 durch Schwenken der Führungshülse 102 um den Führungsstab 101 so ausgerichtet werden, dass seine Längsachse mit der Achse 107 des Innengewindes 29 zusammenfällt.

Zum Einsetzen der Pfanne 4 in den Grundkörper 1 wird zunächst der Führungsstab 101 in die Bohrung 8" eingeschraubt und die Führungshülse 102 über den Führungsstab 101 gesteckt, um die Achse des Eindrehinstruments 104 im wesentlichen parallel zur Achse 107 des Innengewindes 29 auszurichten. Anschließend wird das Werkzeug 101 um den Führungsstab 101 herum gedreht, bis die Achse des Eindrehinstruments 104 mit der Achse des Innengewindes 29 im wesentlichen zusammenfällt. Daraufhin kann der erste Teil 20 der Pfanne 4 in den Grundkörper 1 eingesetzt und durch eine Drehung des Griffs 121 mit Hilfe des durch die Vorsprünge 23', 24' gebildeten Bajonettverschlusses vorfixiert werden. Danach kann der zweite Teil 21 der Pfanne 4 mit Hilfe des Eindrehwerkzeugs 104 mit dem Grundkörper 1 verschraubt werden. Anschließend kann das Eindrehwerkzeug 104 zurückgezogen und der Adapter 102 vom ersten Teil 20 der Pfanne 4 gelöst werden, und der Führungsstab 101 kann aus der Bohrung 8" herausgeschraubt werden.

Da das Werkzeug 100 eine Ausrichtung des ersten Teils 20 und des zweiten Teils 21 der Pfanne 4 relativ zum Grundkörper 1 ermöglicht, kann der zweite Teil 21 präzise mit dem Grundkörper 1 verschraubt und ein Verklemmen des Außengewindes 26 und des Innengewindes 19, das bei einem freihändigen Einschrauben des zweiten Teils 21 der Pfanne 4 insbesondere in Ausführungsformen der vorliegenden Erfindung auftreten kann, in denen das Außengewinde 26 und das Innengewinde 29 als Feingewinde ausgebildet sind, vermieden werden.

Fig. 12 zeigt eine schematische Perspektivansicht des Grundkörpers 1, nachdem er mit Hilfe von Schrauben (nicht gezeigt) ähnlich den in Fig. 2 und 3 dargestellten Schrauben 7 im Beckenknochen des Patienten (nicht gezeigt) befestigt wurde. Der Grundkörper 1 entspricht dem Grundkörper der Ausführungsform, die oben mit Bezug auf die Fig. 8a bis 10 beschrieben wurde.

In der Öffnung 27 des Grundkörpers 1 ist ein Verschlusselement 40 angebracht, das den zweiten Teil 21 der Pfanne 4, die in der oben mit Bezug auf Fig. 8a bis 10 beschriebenen Ausführungsform verwendet wird, ersetzt. Das Verschlusselement 40 kann am Grundkörper 1 befestigt sein. Zu diesem Zweck kann das Verschlusselement 40 ein Außengewinde aufweisen, das mit dem Innengewinde 29 in der Öffnung 27 des Grundkörpers 1 verschraubt sein kann.

Die dem Aufnahmebereich 3 zugewandte Seite des Verschlusselements 40 weist eine Form auf, die - ebenso wie die Innenform des Aufnahmebereichs 3 - zu dem Lagereinsatz der Hüftgelenksprothese komplementär ist. Beispielsweise kann in Ausführungsformen der Erfindung, in denen der Aufnahmebereich 3 als zweiseitig offene Kugelschale ausgebildet ist, das Verschlusselement 40 die Form einer Kugelschale haben, so dass das Verschlusselement 40 den Aufnahmereich 3 zu einer Vertiefung mit einer Innenform in Gestalt einer Kugelschale ergänzt, wenn das Verschlusselement 40 mit dem Aufnahmebereich 3 des Grundkörpers 1 in Deckung gebracht wird. Allgemein kann das Verschlusselement 40 dafür ausgelegt sein, den Aufnahmebereich 3 zu einer Vertiefung mit einer zu dem Lagereinsatz der Hüftgelenksprothese komplementären Innenform zu ergänzen. Dadurch wird eine problemlose Befestigung des Lagereinsatzes mit Hilfe von Zement ermöglicht.

In manchen Ausführungsformen der vorliegenden Erfindung kann das Verschlusselement 40 Öffnungen aufweisen, die mit Schrauben und/oder Stopfen verschlossen werden können. In anderen Ausführungsformen kann das Verschlusselement 40 ohne Öffnungen ausgebildet sein. Vorteilhafterweise kann so ein Kontakt zwischen dem Zement und dem Knochen im Wesentlichen verhindert werden, wodurch nachteilige Einflüsse des Zements auf die Knochensubstanz im Wesentlichen vermieden werden können.

## Patentansprüche

1. Modulares Hüftimplantat mit:
- einem Grundkörper (1) zum Befestigen an einem Beckenknochen (2); und mit
- einer Pfanne (4) zum Aufnehmen einer Hüftgelenkprothese;
- wobei der Grundkörper (1) an dem Beckenknochen (2) befestigbar ist und einen Aufnahmebereich (3) für die Pfanne (4) aufweist;
- wobei die Pfanne (4) eine zu dem Aufnahmebereich (3) komplementäre Außenform aufweist, und die Pfanne (4) in dem Grundkörper (1) befestigbar ist;
- wobei der Grundkörper (1) und die Pfanne (4) Einstellmittel zur variablen Anordnung der Pfanne (4) bezüglich des Grundkörpers (1) umfassen, wobei die Einstellmittel in Grundkörper (1) und Pfanne (4) ausgebildete zusammenwirkende Führungsmittel umfassen, wobei die Führungsmittel eine Nut (15) und einen Vorsprung (16) umfassen, wobei die Nut (15) auf der Innenseite des Aufnahmebereichs (3) angebracht ist und der Vorsprung (16) auf der Außenseite der Pfanne (4) angebracht ist;
- wobei die Pfanne (4) einen Rand (19) aufweist, der in einer Ebene liegt, zu deren Normale (17) eine Normale (18) einer Ebene durch den Vorsprung (16) einen Winkel α zwischen 5° und 20° aufweist;
***dadurch gekennzeichnet,***
- **dass** die Pfanne (4) aus Titan, einer Titanlegierung, einer Titanverbindung, einer Edelstahllegierung oder einer Kobalt-Chrom-Legierung besteht;
- wobei die Pfanne (4) zum Aufnehmen einer künstlichen Gelenkpfanne einer Hüftgelenkprothese, die durch einen Lagereinsatz (30) gebildet wird, ausgelegt ist;
- wobei die Pfanne (4) und der Aufnahmebereich (3) eine Vielzahl von Bohrungen (8, 9) aufweisen, wobei korrespondierende Bohrungen (8, 9) in der Pfanne (4) und dem Aufnahmebereich (3) durch Drehen der Pfanne (4) entlang der Führungsmittel in Flucht miteinander bringbar sind; und
- wobei der Grundkörper (1) einen oberen Rand (50) aufweist, der in einer Ebene liegt, zu deren Normale eine Normale einer Ebene durch die Nut (16) einen Winkel β zwischen 5° und 25° aufweist,
- so dass durch Verdrehen der Pfanne (4) in dem Aufnahmebereich (3) ein Winkel zwischen der Ebene des Randes (19) der Pfanne (4) und der Ebene des Randes (50) des Grundkörpers (1) kontinuierlich einstellbar ist.

2. Hüftimplantat nach Anspruch 1, **dadurch gekennzeichnet, dass** die Vielzahl von Bohrungen in der Pfanne (4) und dem Aufnahmebereich (3) Bohrungen (8, 9) mit unterschiedlichem Durchmesser umfasst.

3. Hüftimplantat nach einem der Ansprüche 1 und 2, **dadurch gekennzeichnet, dass** die Außenseite der Pfanne (4) so ausgebildet ist, dass die Pfanne (4) an dem Grundkörper (1) über eine Zementschicht befestigbar ist.

4. Hüftimplantat nach einem der vorhergehenden Ansprüche, **dadurch gekennzeichnet, dass** die Führungsmittel so angeordnet sind, dass sowohl eine Inklination, als auch eine Anteversion simultan einstellbar sind.

5. Hüftimplantat nach einem der vorhergehenden Ansprüche, **dadurch gekennzeichnet, dass** der Vorsprung (16) oder die Nut (15) ringförmig umlaufend ausgeformt ist.

6. Hüftimplantat nach einem der vorhergehenden Ansprüche, wobei der Winkel α eine Größe zwischen 10° und 12°, vorzugsweise eine Größe von 17°, aufweist.

7. Hüftimplantat nach einem der vorhergehenden Ansprüche, wobei der Winkel β eine Größe zwischen 10° und 22°, vorzugsweise eine Größe von 20°, aufweist.

8. Hüftimplantat nach einem der vorhergehenden Ansprüche, **dadurch gekennzeichnet, dass** das Hüftimplantat eine oder mehrere Bohrungen (8, 9) zum Aufnehmen von den Grundkörper (1) mit dem Beckenknochen verbindenden Schrauben umfasst, die jeweils durch einen Stopfen verschließbar sind.

9. Hüftimplantat nach einem der vorhergehenden Ansprüche, **dadurch gekennzeichnet, dass** der Grundkörper mindestens eine Bohrung umfasst, die ein Innengewinde umfasst, das zur Aufnahme winkelstabiler Schrauben ausgelegt ist.

## Claims

1. Modular hip implant comprising:
- a base member (1) for attachment to a pelvic bone (2); and comprising
- a cup (4) for accommodating a hip joint prosthesis;
- the base member (1) being attachable to the pelvic bone (2) and having an accommodating region (3) for the cup (4);
- the cup (4) having an outer shape which is complementary to the accommodating region (3), and the cup (4) being attachable in the base member (1);
- the base member (1) and the cup (4) comprising adjusting means for variable arrangement of the cup (4) in relation to the base member (1), the adjusting means comprising cooperating guide means formed in the base member (1) and the cup (4), the guide means comprising a groove (15) and a projection (16), the groove (15) being positioned on the inner face of the accommodating region (3) and the projection (16) being positioned on the outer face of the cup (4);
- the cup (4) having a rim (19), which is located in a plane, with whose normal (17) a normal (18) to a plane through the projection (16) includes an angle α of between 5° and 20°; **characterised in that**
- the cup (4) consists of titanium, a titanium alloy, a titanium compound, a stainless steel alloy or a cobalt-chromium alloy;
- the cup (4) being configured to accommodate an artificial joint cup of a hip joint prosthesis which is formed by a bearing insert (30);
- the cup (4) and the accommodating region (3) having a plurality of holes (8, 9), corresponding holes (8, 9) in the cup (4) and in the accommodating region (3) being able to be aligned with one another by rotating the cup (4) along the guide means; and
- the base member (1) having an upper rim (50), which is located in a plane, with whose normal a normal to a plane through the groove (16) includes an angle β of between 5° and 25°,
- in such a way that by rotating the cup (4) in the accommodating region (3), an angle between the plane of the rim (19) of the cup (4) and the plane of the rim (50) of the base member (1) can be continuously adjusted.

2. Hip implant according to claim 1, **characterised in that** the plurality of holes in the cup (4) and in the accommodating region (3) comprises holes (8, 9) of different diameters.

3. Hip implant according to either claim 1 or claim 2, **characterised in that** the outer face of the cup (4) is formed in such a way that the cup (4) can be attached to the base member (1) via a cement layer.

4. Hip implant according to any of the preceding claims, **characterised in that** the guide means are arranged in such a way that both an inclination and an anteversion are simultaneously adjustable.

5. Hip implant according to any of the preceding claims, **characterised in that** the projection (16) or the groove (15) is formed in an annularly circumferential manner.

6. Hip implant according to any of the preceding claims, wherein the size of the angle α is between 10° and 12°, preferably 17°.

7. Hip implant according to any of the preceding claims, wherein the size of the angle β is between 10° and 22°, preferably 20°.

8. Hip implant according to any of the preceding claims, **characterised in that** the hip implant comprises one or a more holes (8, 9) for accommodating the screws which connect the base member (1) to the pelvic bone, each of which holes can be closed by a plug.

9. Hip implant according to any of the preceding claims, **characterised in that** the base member comprises at least one hole, which comprises an internal thread and is configured to accommodate an angularly stable screw.

## Revendications

1. Implant modulaire de la hanche, avec :
- un corps de base (1) à fixer à un os du bassin (2) ; et
- une cavité (4) pour recevoir une prothèse de hanche ;
- étant précisé que le corps de base (1) est apte à être fixé à l'os du bassin (2) et présente une zone de réception (3) pour la cavité (4) ;
- que la cavité (4) présente une forme extérieure complémentaire de la zone de réception (3) et que la cavité (4) est apte à être fixée dans le corps de base (1) ;
- que le corps de base (1) et la cavité (4) comprennent des moyens de réglage pour une disposition variable de la cavité (4) par rapport au corps de base (1), que ces moyens de réglage comprennent des moyens de guidage coopérants qui sont formés dans le corps de base (1) et la cavité (4), que les moyens de guidage comprennent une rainure (15) et une saillie (16), et que la rainure (15) est prévue sur le côté intérieur de la zone de réception (3) tandis que la saillie (16) est prévue sur le côté extérieur de la cavité (4) ;
- que la cavité (4) présente un bord (19) qui est situé dans un plan avec la normale (17) duquel une normale (18) d'un plan traversant la saillie (16) présente un angle α situé entre 5° et 20° ;
***caractérisé en ce que***
- la cavité (4) se compose de titane, d'un alliage de titane, d'un composé du titane, d'un alliage d'acier spécial ou d'un alliage cobalt-chrome ;
- que la cavité (4) est conçue pour recevoir une cavité artificielle d'une prothèse de hanche formée par un insert d'appui (30) ;
- que la cavité (4) et la zone de réception (3) présentent plusieurs perçages (8, 9), et que les perçages correspondants (8, 9) prévus dans la cavité (4) et la zone de réception (3) sont aptes à être alignés grâce à une rotation de la cavité (4) le long des moyens de guidage ; et
- que le corps de base (1) présente un bord supérieur (50) qui est situé dans un plan avec la normale duquel une normale d'un plan traversant la rainure (16) présente un angle β situé entre 5° et 25°,
- de sorte que grâce à une rotation de la cavité (4) dans la zone de réception (3), un angle entre le plan du bord (19) de la cavité (4) et le plan du bord (50) du corps de base (1) est réglable de manière continue.

2. Implant de hanche selon la revendication 1, **caractérisé en ce que** les perçages prévus dans la cavité (4) et la zone de réception (3) comprennent des perçages (8, 9) de diamètres différents.

3. Implant de hanche selon l'une des revendications 1 et 2, **caractérisé en ce que** le côté extérieur de la cavité (4) est conçu de telle sorte que la cavité (4) soit apte à être fixée au corps de base (1) par l'intermédiaire d'une couche de ciment.

4. Implant de hanche selon l'une des revendications précédentes, **caractérisé en ce que** les moyens de guidage sont disposés de telle sorte qu'on puisse régler simultanément aussi bien une inclinaison qu'une antéversion.

5. Implant de hanche selon l'une des revendications précédentes, **caractérisé en ce que** la saillie (16) ou la rainure (15) est formée sur tout le tour suivant une forme annulaire.

6. Implant de hanche selon l'une des revendications précédentes, étant précisé que l'angle α présente une valeur située entre 10° et 12°, de préférence une valeur de 17°.

7. Implant de hanche selon l'une des revendications précédentes, étant précisé que l'angle β présente une valeur située entre 10° et 22°, de préférence une valeur de 20°.

8. Implant de hanche selon l'une des revendications précédentes, **caractérisé en ce que** l'implant de hanche comprend un ou plusieurs perçages (8, 9) qui sont destinés à recevoir des vis reliant le corps de base (1) à l'os du bassin, et qui sont aptes à être fermés chacun par un bouchon.

9. Implant de hanche selon l'une des revendications précédentes, **caractérisé en ce que** le corps de base comprend au moins un perçage qui comprend un filetage intérieur conçu pour recevoir des vis à angle stable.
